(19) Europäisches Patentamt
European Patent Office
Office européen des brevets

(11) Veröffentlichungsnummer: **0 002 260**
**B1**

(12) **EUROPÄISCHE PATENTSCHRIFT**

(45) Veröffentlichungstag der Patentschrift: **14.07.82**

(51) Int. Cl.³: **C 07 D 211/14,** C 07 D 211/58, C 07 D 211/46, C 07 D 211/94, C 08 K 5/34

(21) Anmeldenummer: **78101492.3**

(22) Anmeldetag: **01.12.78**

(54) **Malonsäurederivate von sterisch gehinderten Piperidinen, Verfahren zu ihrer Herstellung und mit ihnen stabilisiertes organisches Material.**

(30) Priorität: **02.12.77 CH 14769/77**

(43) Veröffentlichungstag der Anmeldung: **13.06.79 Patentblatt 79/12**

(45) Bekanntmachung des Hinweises auf die Patenterteilung: **14.07.82 Patentblatt 82/28**

(84) Benannte Vertragsstaaten: **BE CH DE FR GB IT NL**

(56) Entgegenhaltungen:
**DE-A-2 456 864**
**DE-A-2 647 452**
**DE-A-2 718 458**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Patentabteilung Postfach, CH-4002 Basel (CH)**

(72) Erfinder: **Rody, Jean, Dr., Rütiring 82, D-4125 Riehen (CH)**
Erfinder: **Karrer, Friedrich, Dr., Rebbergstrasse 5, CH-4800 Zofingen (CH)**

(74) Vertreter: **Zumstein sen., Fritz, Dr. et al, Bräuhausstrasse 4, D-8000 München 2 (DE)**

**EP 0 002 260 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Malonsäurederivate von sterisch gehinderten Piperidinen, Verfahren zu ihrer Herstellung und mit ihnen stabilisiertes organisches Material

Die vorliegende Erfindung betrifft neue Molonate, Verfahren zu ihrer Herstellung, ihre Verwendung als Stabilisatoren, sowie mit ihrer Hilfe gegen lichtinduzierten Abbau stabilisiertes organisches Material.

Malonate von sterisch gehinderten 4-Hydroxypiperidinen sind aus der US-A-3 640 928 und der GB-A-1 399 239 als Stabilisatoren für synthetische Polymere bekannt. Diese Stabilisatoren haben Eigenschaften, die sich bei der technischen Anwendung störend bemerkbar machen, so hinsichtlich Hydrolysestabilität, Flüchtigkeit, Extraktionsbeständigkeit und Exsudationsbeständigkeit. Ferner sind aus der DE-A-2 456 864 sterisch gehinderte Hydroxybenzyl-malonate von sterisch gehinderten 4-Hydroxy-piperidinen als Stabilisatoren für synthetische Polymere bekannt. Zudem sind in DE-A-2 647 452 Alkylhydroxybenzyl-Malonester von sterisch gehinderten Piperidinen beschrieben. Schließlich sind aus DE-A-2 718 458 Malonester von sterisch gehinderten Piperidinen bekannt, in denen beide Hydroxybenzylgruppen ersetzt sind, insbesondere durch Alkyl, Alkenyl oder Benzyl. Auch diese Malonesterderivate dienen als Stabilisatoren für synthetische Polymere. Bei der praktischen Anwendung dieser Stabilisatoren treten jedoch bei thermischer Überbeanspruchung, die auch unbeabsichtigt bei der Einarbeitung oder Verarbeitung auftreten kann, oder etwa bei Zumischung als Schmelze über eine Schnecke in den Extruder, Verfärbungen auf, die vielfach unerwünscht sind.

Ausgehend von diesem Stand der Technik war es Aufgabe der Erfindung, Stabilisatoren für organisches Material zu schaffen, die die Nachteile der bisher bekannten Stabilisatoren nicht oder nur in wesentlich geringerem Maße haben.

Die Erfindung betrifft Malonsäurederivate der Formel I und deren Salze

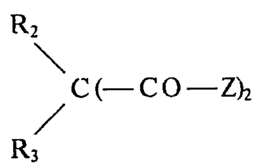

(I)

worin Z eine Gruppe der Formel II oder III ist

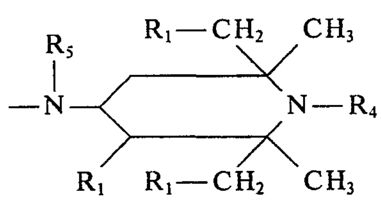

(II)

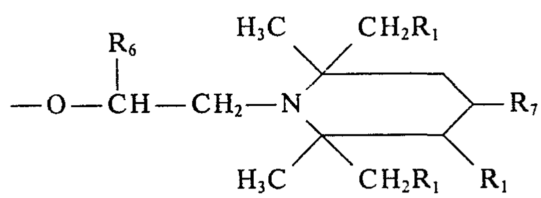

(III)

$R_1$ Wasserstoff oder $C_1-C_4$ Alkyl ist,

$R_2$ $C_1-C_{12}$ Alkyl, $C_3-C_4$ Alkenyl, Benzyl oder gegebenenfalls durch $C_1-C_8$ Alkyl oder $C_1-C_8$ Alkoxy substituiertes Phenyl oder Cyano ist,

$R_3$ $C_1-C_{12}$ Alkyl, $C_3-C_4$ Alkenyl oder Benzyl ist,

$R_4$ Wasserstoff, $O^{\cdot}$, —OH, Alkyl mit 1—12 C-Atomen, Alkenyl mit 3 oder 4 C-Atomen, Propargyl, Benzyl oder eine Gruppe der Formel $-CH_2-CH(OR_9)-R_8$ darstellt, worin $R_8$ Wasserstoff, Methyl oder Phenyl und $R_9$ Wasserstoff oder eine Gruppe A—CO— bedeuten; oder $R_4$ eine Gruppe A—CO— bedeutet und in beiden Fällen A Alkyl mit 1—18 C-Atomen, Alkenyl mit 2 oder 3 C-Atomen, Cyclohexyl, Phenyl, Benzyl, eine durch 2 Alkylgruppen mit je 1—4 C-Atomen und eine Hydroxylgruppe substituierte Phenyl-, Phenylmethyl- oder Phenyläthylgruppe, Alkylamino mit 1—12 C-Atomen, Dialkylamino mit 2—16 C-Atomen, Anilino, Alkoxy mit 1—12 C-Atomen, Benzyloxy oder Phenoxy bedeutet,

$R_5$ Wasserstoff, Alkyl mit 1—18 C-Atomen, Alkenyl mit 3—4 C-Atomen, Propargyl, Cycloalkyl mit 5—12 C-Atomen, Aralkyl mit 7—14 C-Atomen, das durch OH substituiert sein kann, oder eine Gruppe der Formel IV

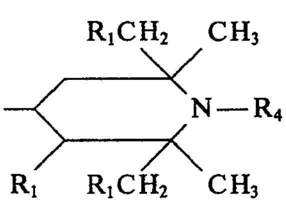

(IV)

$R_6$ Wasserstoff, Methyl, Äthyl oder Phenyl ist,

$R_7$ Wasserstoff, $-OR_{10}$ oder $-N(R_{11})(R_{12})$ und $R_{10}$ Alkyl mit 1—18 C-Atomen, Alkenyl mit 3—4 C-Atomen, Benzyl, 2-Cyanoäthyl, eine aliphatische, cycloaliphatische, aromatische oder araliphatische Acylgruppe mit bis zu 18 C-Atomen, die im aromatischen Teil durch Halogen, Alkoxy mit 1—4 C-Atomen, Alkyl mit 1—4 C-Atomen und/oder Hydroxyl substituiert sein kann, bedeutet, $R_{11}$ Alkyl mit 1—18 C-Atomen, Alkenyl mit 3—4 C-Atomen oder Phenylalkyl mit 7—9 C-Atomen ist und $R_{12}$ dieselbe Bedeutung hat wie $R_{11}$ oder eine aliphatische, cycloaliphatische, aromatische oder araliphatische Acylgruppe mit bis zu 18 C-Atomen darstellt, die im aromatischen Teil durch Halogen, Alkoxy mit 1—4 C-Atomen, Alkyl mit 1—4 C-Atomen und/oder Hydroxyl substituiert sein kann.

$R_1$ ist als $C_1-C_4$-Alkyl verzweigtes oder insbesondere unverzweigtes Alkyl, wie Äthyl, n-Propyl oder n-Butyl, vor allem aber Methyl. Bevorzugt ist $R_1$ Wasserstoff. Alle Substituenten $R_1$ sind gleich.

$R_2$ bzw. $R_3$ ist als $C_1-C_{12}$ Alkyl verzweigtes oder unverzweigtes Alkyl, insbesondere solches mit 2—8 C-Atomen, wie Äthyl, n- oder i-Propyl, n- oder i-Butyl, ein Pentyl, Hexyl, Heptyl oder Octyl, wie n- oder i-Octyl. Gleiches gilt für $R_4$ als Alkyl, $R_5$, A, $R_{10}$ und $R_{11}$ als Alkyl können zudem noch z. B. Tridecyl, Hexadecyl oder Octadecyl sein.

$R_2$ bzw. $R_3$ ist als $C_3-C_4$ Alkenyl insbesondere Methallyl, vor allem Allyl. Gleiches gilt für $R_4$, $R_5$, $R_{10}$ und $R_{11}$. Alkenyl A ist insbesondere Vinyl oder Allyl.

$R_5$ ist als durch $C_1-C_8$ Alkyl substituiertes Phenyl insbesondere solches, das durch Äthyl, n- oder i-Propyl, vor allem aber durch Methyl substituiert ist und als durch $C_1-C_8$ Alkoxy substituiertes Phenyl solches, das durch Äthoxy, n- oder i-Propoxy, vor allem aber durch Methoxy substituiert ist. Bevorzugt ist Phenyl $R_2$ aber unsubstituiert.

Sind $R_2$ und $R_3$ Alkyl, so sollten nicht beide zugleich ein tertiäres $\alpha$-C-Atom aufweisen. So ist im Fall von $R_2$ und $R_3$ gleich Alkyl mindestens eines davon ein solches mit einem primären oder sekundären $\alpha$-C-Atom.

$R_{11}$ und $R_{12}$ sind als Phenylalkyl mit 7—9 C-Atomen z. B. Benzyl, Phenyläthyl oder Phenylpropyl. $R_5$ als Aralkyl mit 7—14 C-Atomen kann darüber hinaus auch z. B. Diphenylmethyl, Naphthylpropyl oder Diphenylmethyl sein.

Wenn $R_4$ und/oder $R_9$ eine Gruppe A—CO— darstellt, so kann dies, je nach der Bedeutung von A, ein Carbonsäurerest sein wie z. B. Acetyl, Propionyl, Butyryl, Capronyl, Capryloyl, Lauroyl, Acryloyl, Crotonoyl, Phenylacetyl-, $\beta$-(3,5-Di-tert.-butyl-4-hydroxyphenyl)-propionyl und -acetyl oder Benzoyl; oder ein Carbamoylrest wie z. B. Methylcarbamoyl, Butylcarbamoyl, Dodecylcarbamoyl, Diäthylcarbamoyl, Dihexylcarbamoyl, Dioctylcarbamoyl oder Phenylcarbamoyl; oder ein Carbonesterrest wie z. B. Äthoxycarbonyl-, Isopropoxycarbonyl, 2-Äthylhexyloxycarbonyl, Dodecyloxycarbonyl, Benzyloxycarbonyl oder Phenoxycarbonyl.

Wenn $R_{10}$ und/oder $R_{12}$ eine aliphatische, cycloaliphatische, aromatische oder araliphatische Acylgruppe mit bis zu 18 C-Atomen darstellt, die im aromatischen Rest durch Halogen, Alkoxy, Alkyl oder OH substituiert sein kann, so kann es sich dabei beispielsweise um Acetyl, Propionyl, Isobutyryl, Capronyl, Capryloyl, Lauroyl, Stearoyl, Oleyl, Cyclohexancarbonyl, Benzoyl, 4-Chlorbenzoyl, Toluoyl, 3-Methoxybenzoyl, 2,4-Dichlorbenzoyl, 4-tert.-Butylbenzoyl, Phenylacetyl, 3,5-Di-tert.butyl-4-hydroxybenzoyl oder 4-Chlorphenylpropionyl handeln.

$R_5$ in der Bedeutung von Cycloalkyl mit 5—8 C-Atomen kann z. B. Cyclopentyl, Cyclohexyl oder Cyclooctyl sein. $R_{12}$ als Cycloalkyl kann drüber hinaus auch z. B. Cyclodecyl oder Cyclododecyl sein.

Bevorzugt sind Malonate Ia der Formel I, worin

| | |
|---|---|
| $R_1$ | Wasserstoff oder Methyl ist, |
| $R_2$ | $C_1-C_8$ Alkyl, Allyl, Methallyl, Benzyl oder Phenyl ist, |
| $R_3$ | $C_2-C_8$ Alkyl, Allyl, Methallyl oder Benzyl ist, |
| Z | eine Gruppe der Formel II oder III ist, |
| $R_4$ | Wasserstoff, $O^{\cdot}$, Alkyl mit 1—8 C-Atomen, Allyl, Propargyl, Benzyl, Formyl, Acetyl, Acryloyl oder Crotonoyl, |
| $R_5$ | Wasserstoff, Alkyl mit 1—12 C-Atomen, Cyclohexyl, Benzyl oder Phenyl, |
| $R_6$ | Wasserstoff, Methyl oder Äthyl, |
| $R_7$ | Wasserstoff oder $-OR_{10}$ und $R_{10}$ Alkyl mit 1—6 C-Atomen, Alkanoyl mit 2—12 C-Atomen, Benzoyl oder Benzyl darstellt. |

Besonders bevorzugt sind Malonate Ib der Formel I, worin

| | |
|---|---|
| $R_1$ | Wasserstoff oder Methyl ist, |
| $R_2$ | $C_1-C_8$ Alkyl, Allyl, Methallyl, Benzyl oder Phenyl ist, |
| $R_3$ | $C_2-C_8$ Alkyl oder Benzyl ist, |
| Z | eine Gruppe der Formel II oder III ist, |
| $R_4$ | Wasserstoff, Methyl, Benzyl oder Acetyl ist, |

| | |
|---|---|
| $R_5$ | Wasserstoff oder $C_1-C_4$ Alkyl ist, und |
| $R_6$ und $R_7$ | Wasserstoff sind. |

Vor allem betrifft die Erfindung Malonate Ic der Formel I, worin

| | |
|---|---|
| $R_1$ | Wasserstoff oder Methyl ist, |
| $R_2$ | $C_1-C_6$ Alkyl, Allyl, Methallyl oder Benzyl ist, |
| $R_3$ | Benzyl ist, und |
| Z | eine Gruppe der Formel II oder III ist, |
| $R_4$ | Wasserstoff oder Methyl ist, und |
| $R_5$, $R_6$ und $R_7$ | Wasserstoff sind. |

Bevorzugt ist in obigen Malonaten I und Ia-Ic $R_2$ und $R_3$ Benzyl. Bevorzugt ist zudem $R_1$ Wasserstoff.

Beispiele für Malonate der Formel I sind aus den Ausführungsbeispielen ersichtlich. Diese sind vor allem bevorzugt.

Die vorliegende Erfindung umfaßt auch die Salze von Verbindungen der Formel I, die durch Addition von Säuren in maximal den Piperidingruppen äquivalenten Mengen entstehen. Solche Säuren können anorganische Säuren sein, wie z. B. Schwefel-, Salz oder Phosphorsäure, organische Carbonsäuren wie Ameisen-, Essig-, Oxal-, Malein-, Benzoe- oder Salicylsäure, organische Sulfonsäuren wie Methan- oder p-Toluolsulfonsäure oder organische phosphorhaltige Säuren, wie Diphenylphosphorsäure, Methanphosphonsäure oder Diphenylphosphinsäure.

Die Verbindungen der Formel I können nach verschiedenen mehrstufigen Verfahren hergestellt werden, wobei die einzelnen Verfahrensschritte an sich bekannte Reaktionen darstellen.

Zur Herstellung der Verbindungen der Formel I geht man vorzugsweise von Verbindungen der Formel $CH_2(-COZ)_2$ aus. In diese führt man entweder zuerst den Substituenten $R_2$ und anschließend den Substituenten $R_3$ ein oder man führt zuerst den Substituenten $R_3$ und anschließend $R_2$ ein. Sind $R_2$ und $R_3$ identisch, so können beide in einem Schritt eingeführt werden.

Die Ausgangsmaterialien $CH_2(COZ)_2$ können durch Umsetzung der entsprechenden Niederalkylester $CH_2$(COO-Alkyl)$_2$ mit den Piperidinderivaten der Formel ZH hergestellt werden. Beispiele für letztere Verbindungen sind 1,2,2,6,6-Pentamethyl-4-aminopiperidin, 1-Hydroxyäthyl-2,2,6,6-Tetramethyl-piperidin, 1-(2-Hydroxypropyl)-2,2,6,6-tetramethyl-piperidin, oder 1-(2-Hydroxypropyl)-2,3,6-trimethyl-2,6-diäthyl-4-acetoxypiperidin. Anstelle der Niederalkylester können auch die entsprechenden Säurechloride $CH_2(COCl)_2$ mit den Piperidinderivaten umgesetzt werden.

Der Rest $R_2$ kann nach Art einer Malonestersynthese eingeführt werden, indem eine Verbindung $CH_2(-COZ)_2$ erst durch Reaktion mit einem Äquivalent Alkalimetall, Alkalialkoholat, Alkaliamid, Alkalihydrid oder einer ähnlichen basischen Alkaliverbindung in die Alkaliverbindung übergeführt wird und anschließend mit 1 Mol eines $R_2$-Halogenides $R_2$ Hal (Hal = Cl, Br oder J) in üblicher Weise umgesetzt wird.

Anschließend muß in dieses $R_2$-Malonsäurederivat der Substituent $R_3$ eingeführt werden. Ist allerdings $R_2$ gleich $R_3$, so kann die Einführung beider Reste vorteilhaft gleichzeitig erfolgen.

Die Einführung des Substituenten $R_3$ kann nach der klassischen Methode der C-Alkylierung von Malonsäurederivaten geschehen, wobei wiederum zunächst die Alkaliverbindung hergestellt und dann mit einer Halogenverbindung $R_3$Hal umgesetzt wird. Hierbei bedeutet Hal wieder Cl, Br oder J. Pro Mol Alkaliverbindung wird etwa ein Mol eines Monohalogenides $R_3$Hal verwendet. Beispiele hierfür sind Alkyl-, Alkenyl- oder Benzylhalogenide.

Schließlich kann auch die Einführung von $R_4$ zusammen mit der Einführung von $R_3$ geschehen, wenn $R_4$ und $R_3$ identisch sind, z. B. in ihrer Bedeutung als Alkyl, Alkenyl oder Benzyl.

Auf Grund dieser mannigfachen Möglichkeiten zur Durchführung der einzelnen Reaktionsschritte,

Einführung des Piperidinylrestes,
Einführung der Gruppe $R_2$,
Einführung der Gruppe $R_3$,
und gegebenenfalls Einführung von $R_4$,

wird man die Reihenfolge der einzelnen Schritte so wählen, wie es im einzelnen Fall am zweckmäßigsten erscheint.

Vorteilhaft kann man obige Umsetzung eines Malonsäureniederalkylesters mit einem Piperidinderivat auch mit einem Malonester durchführen, der in $\alpha$-Stellung durch $R_2$ und $R_3$ substituiert ist, wobei man insbesondere wie oben vorgeht.

Die Ausgangsstoffe sind bekannt oder können, sofern sie neu sind, nach an sich bekannten Methoden und in Analogie zu bekannten Verbindungen hergestellt werden.

Die Verbindungen der Formel I können gemäß der vorliegenden Erfindung als Stabilisatoren für Kunststoffe gegen deren Schädigung durch Einwirkung von Sauerstoff, Wärme und Licht verwendet werden. Beispiele für solche Kunststoffe sind Polyäther-polyester, sowie die in der DE-A-2 456 864 auf den Seiten 12—14 aufgeführten Polymeren.

Von besonderer Bedeutung ist die Stabilisierung von Polyolefinen, Styrolpolymerisaten und vom Polyurethanen, für die sich die Verbindungen der Formel I hervorragend eignen. Beispiele hierfür sind Polyäthylen hoher und niedriger Dichte. Polypropylen, Äthylen-Polypropylen-Copolymerisate, Polystyrol, Styrol-Butadien-Acrylnitril-Copolymerisate, Mischungen von Polyolefinen oder von Styrolpolymerisaten, Polyurethane auf Polyäther- oder Polyesterbasis in Form von Lacken, Elastomeren oder Schaumstoffen.

Die Stabilisatoren werden den Kunststoffen in einer Konzentration von 0,01 bis 5 Gew.-% berechnet auf das zu stabilisierende Material, zugesetzt. Vorzugsweise werden 0,03 bis 1,5, besonders bevorzugt 0,2 bis 0,6 Gew.-% der Verbindungen, berechnet auf das zu stabilisierende Material, in dieses eingearbeitet.

Die Einarbeitung kann nach der Polymerisation erfolgen, beispielsweise durch Einmischen der Verbindungen und gegebenenfalls weiterer Additive in die Schmelze nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels.

Die neuen Verbindungen können auch in Form eines Masterbatches, der diese Verbindungen beispielsweise in einer Konzentration von 2,5 bis 25 Gew.-% enthält, den zu stabilisierenden Kunststoffen zugesetzt werden.

Im Falle von vernetztem Polyäthylen werden die Verbindungen vor der Vernetzung beigefügt.

Außer den Verbindungen der Formel I können den Kunststoffen auch noch andere, bekannte Stabilisatoren zugesetzt werden. Dies können z. B. Antioxydantien, Lichtschutzmittel oder Metalldesaktivatoren sein, oder auch Costabilisatoren wie z. B. solche vom Typ der Phosphorigsäureester. Weiterhin können sonstige in der Kunststofftechnologie übliche Zusätze wie z. B. Flammschutzmittel, Antistatika, Weichmacher, Gleitmittel, Treibmittel, Pigmente, Verstärkungsstoffe oder Füllstoffe zugesetzt werden.

Die Erfindung betrifft daher auch die durch Zusatz von 0,01 bis 5 Gew.-% einer Verbindung der Formel I stabilisierten Kunststoffe, die gegebenenfalls noch andere bekannte und übliche Zusätze enthalten können. Die so stabilisierten Kunststoffe können in verschiedenster Form angewendet werden, z. B. als Folien, Fasern, Bändchen, Profile oder als Bindemittel für Lacke, Klebemittel oder Kitte.

Die Herstellung und Verwendung der erfindungsgemäßen Verbindungen wird in den folgenden Beispielen näher beschrieben. Teile bedeuten darin Gewichtsteile und % Gewichtsprozente. Die Temperaturen sind in Celsius-Graden angegeben.

## Beispiel 1

Eine auf 125° erhitzte Lösung von 31,2 g Dibenzylmalonsäure-dimethylester (Smp. 60—61°) und 37,1 g 1-(2-Hydroxyäthyl)-2,2,6,6-tetramethyl-piperidin (Smp. 96—97°) in 300 ml wasserfreiem Xylol versetzt man unter Rühren mit 0,15 g Lithiumamid. Hierauf wird das Reaktionsgemisch unter gleichzeitigem Durchleiten eines schwachen Stickstoffstromes innerhalb von 3 Stunden bis auf 136° C erhitzt und über einen absteigenden Kühler das freigesetzte Methanol sowie auch Xylol abdestilliert. Die Innentemperatur wird dann auf 150° C gesteigert und das Xylol weitgehend abdestilliert (Dauer ca. 4 Stunden). Nach dem Abkühlen wird das Reaktionsgemisch in Chloroform gelöst, wiederholt mit Wasser gewaschen, über Natriumsulfat getrocknet das Lösungsmittel im Vakuum vollständig abdestilliert und der Rückstand in n-Hexan kristallisiert, wodurch reiner Dibenzylmalonsäure-bis-[2-(2,2,6,6-tetramethyl-1-piperidinyl)-äthyl]-ester vom Smp. 77—78° C erhalten wurde (Stabilisator Nr. 1).

## Beispiel 2

In analoger Weise wie nach Beispiel 1 erhält man aus Dibenzylmalonsäure-dimethylester und 1-(2-Hydroxy-propyl)-2,2,6,6-tetramethyl-piperidin den Dibenzylmalonsäure-bis-[2-(2,2,6,6-tetramethyl-1-piperidinyl)-propyl]-ester (Diastereoisomerengemisch), Smp. etwa 85° (Stabilisator Nr. 2).

| | | | |
|---|---|---|---|
| Berechnet: | C 76,1, | H 9,66, | N 4,33%; |
| Gefunden: | C 75,9, | H 9,6, | N 4,5%. |

## Beispiel 3

In analoger Weise wie nach Beispiel 1 erhält man aus Dibenzylmalonsäure-dimethylester und 1-(2-Hydroxy-2-phenyl-äthyl)-2,2,6,6-tetramethyl-piperidin den Dibenzylmalonsäure-bis-[1-phenyl-2-(2,2,6,6-tetramethyl-1-piperidinyl)-äthyl]-ester (Diastereoisomerengemisch), Smp. etwa 140° (Stabilisator Nr. 3).

| | | | |
|---|---|---|---|
| Berechnet: | C 79,23, | H 8,87, | N 3,62%; |
| Gefunden: | C 79,4, | H 8,6, | N 3,6%. |

## Beispiel 4

Zu einer Suspension von ölfreiem Natriumhydrid (0,97 g) in Toluol (20 ml) tropft man unter Rühren innerhalb von ca. 1/2 Stunde bei 40° die Lösung von 20 g Benzylmalonsäure-bis-(1,2,2,6,6-pentamethyl-4-piperidinyl)-amid in 50 ml Toluol und 100 ml Dimethylformamid. Nach der Zugabe des Amids wird das Reaktionsgemisch für weitere 20 Stunden bei 60° C gerührt bis alles Natriumhydrid umgesetzt ist. Hierauf tropft man innerhalb von 40 Minuten bei Raumtemperatur die Lösung von 7,7 g Benzylbromid in 10 ml Toluol zum Reaktionsgemisch, erhöht anschließend die Temperatur auf 70° und rührt weitere 12 Stunden bei dieser Temperatur. Zur Aufarbeitung wird die Reaktionslösung warm vom ausgefallenen Natriumbromid abfiltriert, der Rückstand mit Chloroform gut gewaschen und die vereinigten Filtrate am Rotationsverdampfer möglichst vollständig von allen Lösungsmitteln befreit. Den Rückstand löst man in 400 ml Chloroform, extrahiert die Lösung wiederholt mit Wasser, trocknet über Natriumsulfat und entfernt das Lösungsmittel im Vakuum. Der kristalline Rückstand wird einmal in Toluol und einmal in Benzol umkristallisiert, wodurch Dibenzylmalonsäure-bis-(1,2,2,6,6-pentamethyl-4-piperidinyl)-amid vom Smp. 275—277° erhalten wurde (Stabilisator Nr. 4).

Das als Ausgangsmaterial verwendete Benzylmalonsäure-bis-(1,2,2,6,6-pentamethyl-4-piperidinyl)-amid läßt sich durch Amidierung von Benzylmalonsäure-diäthylester mit mindestens 2 Äquivalenten 4-Amino-1,2,2,6,6-pentamethyl-piperidin bei 130—150° in Gegenwart eines basischen Katalysators, wie z. B. Lithiumamid, herstellen, Smp. 242—243°.

## Beispiel 5

In analoger Weise wie nach Beispiel 4 erhält man aus Benzylmalonsäure-bis-(1,2,2,6,6-pentamethyl-4-piperidinyl)-amid und n-Octylbromid das Benzyl-n-octyl-bis-(1,2,2,6,6-pentamethyl-4-piperidinyl)-amid, Smp. 164—166° (Stabilisator Nr. 5).

## Beispiel 6

100 Teile Polypropylenpulver (Moplen, Fibre grade, der Firma Montedison) werden mit 0,2 Teilen β-(3,5-Di-tert.butyl-4-hydroxyphenyl)-propionsäure-octadecylester und 0,25 Teilen eines Stabilisators der folgenden Tabelle I im Brabenderplastographen bei 200° C während 10 Minuten homogenisiert. Die so erhaltene Masse wird möglichst rasch dem Kneter entnommen und in einer Kniehebelpresse zu einer 2—3 mm dicken Platte gepreßt. Ein Teil des erhaltenen Rohpreßlings wird ausgeschnitten und zwischen zwei Hochglanz-Hartaluminiumfolien mit einer Laborpresse während 6 Minuten bei 260° und 40 kg/cm² Druck zu einer 0,1 mm dicken Folie gepreßt, die unverzüglich in kaltem Wasser abgeschreckt wird. Aus dieser werden nun Abschnitte von je 60 × 44 mm gestanzt und mit der in der Tabelle I jeweils erwähnten Belichtungsapparatur belichtet. In regelmäßigen Zeitabständen werden diese Prüflinge aus dem Belichtungsapparat entnommen und in einem IR-Spektrophotometer auf ihren Carbonylgehalt geprüft. Die Zunahme der Carbonylextinktion bei 5.85 μ bei der Belichtung ist ein Maß für den photooxidativen Abbau des Polymeren (s. L. Balaban et al., H. Polymer Sci. Part C, 22, 1059—1071 (1969)) und ist erfahrungsgemäß mit einem Abfall der mechanischen Eigenschaften des Polymeren verbunden. So ist z. B. die Folie bei Erreichen einer Carbonylextinktion von ca. 0,300 vollständig brüchig.

Die Schutzwirkung der Stabilisatoren gemäß Erfindung ist aus folgender Tabelle I ersichtlich:

Tabelle 1

| Stabilisator Nr. | Belichtungszeit in Stunden bis CO-Extinktion = 0,3 | Xenotest |
|---|---|---|
| 1 | 4370 | 1200 |
| 2 | 2395 | 1200 |
| 3 | 3765 | 1200 |
| Kontrolle | ~ 555 | 1200 |

Beispiel 7

In analoger Weise wie nach Beispiel 1 erhält man aus Dibenzylmalonsäuredimethylester und 1-(2-Hydroxy-butyl)-2,2,6,6-tetramethyl-piperidin (Smp.: 109—110° C) den Dibenzylmalonsäure-bis-[2-(2,2,6,6-tetramethyl-1-piperidinyl)-butyl]-ester (Diastereoisomerengemisch) als farblosen Harz.

Analyse:
Berechnet: C 76,51, H 9,86, N 4,15%;
Gefunden: C 76,7, H 10,1, N 4,2%.

Das $^1$H-NMR-Spektrum stimmt mit der Struktur der Verbindung gut überein.

**Patentansprüche**

1. Malonsäurederivate der Formel I und deren Salze

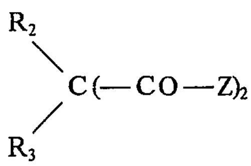

(I)

worin Z eine Gruppe der Formel II oder III ist

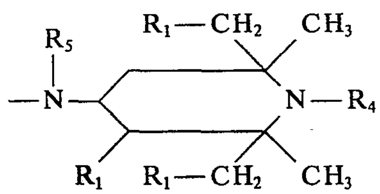

(II)

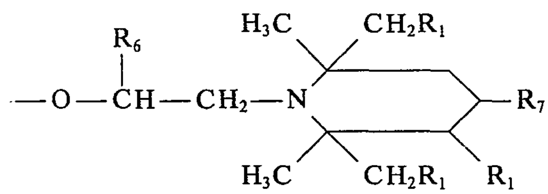

(III)

$R_1$ Wasserstoff oder $C_1$—$C_4$ Alkyl ist,
$R_2$ $C_1$—$C_{12}$ Alkyl, $C_3$—$C_4$ Alkenyl, Benzyl oder gegebenenfalls durch $C_1$—$C_8$ Alkyl oder $C_1$—$C_8$ Alkoxy substituiertes Phenyl oder Cyano ist,
$R_3$ $C_1$—$C_{12}$ Alkyl, $C_3$—$C_4$ Alkenyl oder Benzyl ist,
$R_4$ Wasserstoff, $O^{\cdot}$, —OH, Alkyl mit 1—12 C-Atomen, Alkenyl mit 3 oder 4 C-Atomen, Propargyl, Benzyl oder eine Gruppe der Formel $-CH_2-CH(OR_9)-R_8$ darstellt, worin $R_8$ Wasserstoff, Methyl oder Phenyl und $R_9$ Wasserstoff oder eine Gruppe A—CO— bedeuten; oder $R_4$ eine Gruppe A—CO— bedeutet und in beiden Fällen A Alkyl mit 1—18 C-Atomen, Alkenyl mit 2 oder 3 C-Atomen, Cyclohexyl, Phenyl, Benzyl, eine durch 2 Alkylgruppen mit je 1—4 C-Atomen und eine Hydroxylgruppe substituierte Phenyl-, Phenylmethyl- oder Phenyläthylgruppe, Alkylamino mit 1—12 C-Atomen, Dialkylamino mit 2—16 C-Atomen, Anilino, Alkoxy mit 1—12 C-Atomen, Benzyloxy oder Phenoxy bedeutet,
$R_5$ Wasserstoff, Alkyl mit 1—18 C-Atomen, Alkenyl mit 3—4 C-Atomen, Propargyl, Cycloalkyl mit 5—12 C-Atomen, Aralkyl mit 7—14 C-Atomen, das durch OH substituiert sein kann, oder eine Gruppe der Formel IV

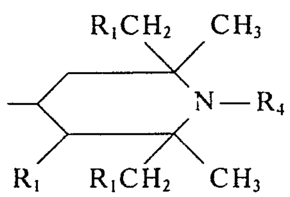

(IV)

bedeutet

$R_6$ Wasserstoff, Methyl, Äthyl oder Phenyl ist,

$R_7$ Wasserstoff, $-OR_{10}$ oder $-N(R_{11})(R_{12})$ und $R_{10}$ Alkyl mit 1—18 C-Atomen, Alkenyl mit 3—4 C-Atomen, Benzyl, 2-Cyanoäthyl, eine aliphatische, cycloaliphatische, aromatische oder araliphatische Acylgruppe mit bis zu 18 C-Atomen, die im aromatischen Teil durch Halogen, Alkoxy mit 1—4 C-Atomen, Alkyl mit 1—4 C-Atomen und/oder Hydroxyl substituiert sein kann, bedeutet, $R_{11}$ Alkyl mit 1—18 C-Atomen, Alkenyl mit 3—4 C-Atomen oder Phenylalkyl mit 7—9 C-Atomen ist und $R_{12}$ dieselbe Bedeutung hat wie $R_{11}$ oder eine aliphatische, cycloaliphatische, aromatische oder araliphatische Acylgruppe mit bis zu 18 C-Atomen darstellt, die im aromatischen Teil durch Halogen, Alkoxy mit 1—4 C-Atomen, Alkyl mit 1—4 C-Atomen und/oder Hydroxyl substituiert sein kann.

2. Malonsäurederivate nach Anspruch 1, worin

$R_1$ Wasserstoff oder Methyl ist,
$R_2$ $C_1-C_8$-Alkyl, Allyl, Methallyl, Benzyl oder Phenyl ist,
$R_3$ $C_2-C_8$ Alkyl, Allyl, Methallyl oder Benzyl ist,
Z eine Gruppe der Formel II oder III ist,
$R_4$ Wasserstoff, O˙, Alkyl mit 1—8 C-Atomen, Allyl, Propargyl, Benzyl, Formyl, Acetyl, Acryloyl oder Crotonoyl,
$R_5$ Wasserstoff, Alkyl mit 1—12 C-Atomen, Cyclohexyl, Benzyl oder Phenyl,
$R_6$ Wasserstoff, Methyl oder Äthyl,
$R_7$ Wasserstoff oder $-OR_{10}$ und $R_{10}$ Alkyl mit 1—6 C-Atomen, Alkanoyl mit 2—12 C-Atomen, Benzoyl oder Benzyl darstellt.

3. Malonsäurederivate nach Anspruch 1, worin

$R_1$ Wasserstoff oder Methyl ist,
$R_2$ $C_1-C_8$ Alkyl, Allyl, Methallyl, Benzyl oder Phenyl ist,
$R_3$ $C_2-C_8$ Alkyl oder Benzyl ist,
Z eine Gruppe der Formel II oder III ist,
$R_4$ Wasserstoff, Methyl, Benzyl oder Acetyl ist,
$R_5$ Wasserstoff oder $C_1-C_4$ Alkyl ist, und
$R_6$ und $R_7$ Wasserstoff sind.

4. Malonsäurederivate nach Anspruch 1, worin

$R_1$ Wasserstoff oder Methyl ist,
$R_2$ $C_1-C_6$ Alkyl, Allyl, Methallyl oder Benzyl ist,
$R_3$ Benzyl ist, und
Z eine Gruppe der Formel II oder III ist
$R_4$ Wasserstoff oder Methyl ist, und
$R_5$, $R_6$ und $R_7$ Wasserstoff sind.

5. Malonsäurederivat nach Anspruch 1, nämlich Dibenzylmalonsäure-bis-[2-(2,2,6,6-tetramethyl-1-piperidinyl)-äther]-ester.

6. Stabilisiertes organisches Material enthaltend eines der in einem der Ansprüche 1 bis 5 genannten Malonsäurederivate.

7. Stabilisiertes organisches Material nach Anspruch 6, nämlich ein Polyolefin, Styrolpolymer oder Polyurethan.

8. Verfahren zum Stabilisieren von organischem Material, dadurch gekennzeichnet, daß man ein Malonsäurederivat gemäß einem der Ansprüche 1 bis 5 verwendet.

9. Verfahren nach Anspruch 1 zur Herstellung von Malonsäurederivaten oder Salzen davon gemäß einem der Ansprüche 1—5, dadurch gekennzeichnet, daß man in ein Malonsäurederivat der Formel I, worin $R^2$ und/oder $R_3$ Wasserstoff ist, $R_2$ und/oder $R_3$ in an sich bekannter Weise einführt, oder in einem $R_2$, $R_3$ Malonsäuredinideralkylester Niederalkoxy in an sich bekannter Weise gegen den Rest Z austauscht, und wenn erwünscht, in ein Malonsäurederivat der Formel I, worin $R_4$ Wasserstoff ist,

einen Substituenten $R_4$ in an sich bekannter Weise einführt, und/oder eine freie Verbindung in ein Salz, ein Salz in eine freie Verbindung oder ein Salz in ein anderes Salz umwandelt.

**Claims**

1. A malonic acid derivative of the formula I

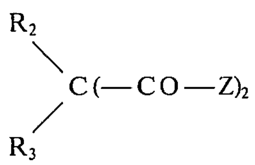

(I)

wherein

Z is a group of the formula II or III

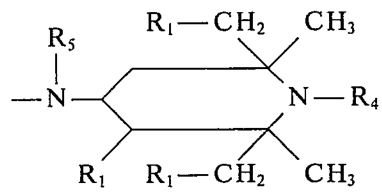

(II)

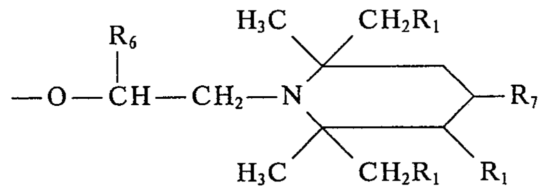

(III)

$R_1$ is hydrogen or $C_1-C_4$alkyl,
$R_2$ is $C_1-C_{12}$alkyl, $C_3-C_4$alkenyl, benzyl, or phenyl which is unsubstituted or substituted by $C_1-C_8$alkyl or $C_1-C_8$alkoxy, or ist cyano,
$R_3$ is $C_1-C_{12}$alkyl, $C_3-C_4$alkenyl or benzyl,
$R_4$ is hydrogen, $O^{\cdot}$, $-OH$, alkyl of 1 to 12 carbon atoms, alkenyl of 3 or 4 carbon atoms, propargyl, benzyl or a group of the formula $-CH_2-CH(OR_9)-R_8$, wherein $R_8$ is hydrogen, methyl, or phenyl, and $R_9$ is hydrogen or a group $A-CO-$; or $R_4$ is a group $A-CO-$ and in both cases A is alkyl of 1 to 18 carbon atoms, alkenyl of 2 or 3 carbon atoms, cyclohexyl, phenyl, benzyl, a phenyl, phenylmethyl or phenylethyl group which is substituted by 2 alkyl groups, each containing 1 to 4 carbon atoms, and by a hydroxyl group, or is alkylamino of 1 to 12 carbon atoms, dialkylamino of 2 to 16 carbon atoms, anilino, alkoxy of 1 to 12 carbon atoms, benzyloxy or phenoxy,
$R_5$ is hydrogen, alkyl of 1 to 18 carbon atoms, alkenyl of 3 to 4 carbon atoms, propargyl, cycloalkyl of 5 to 12 carbon atoms, aralkyl of 7 to 14 carbon atoms which can be substituted by OH, or is a group of the formula IV

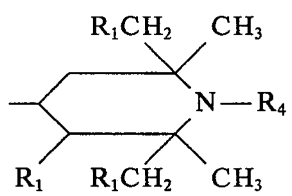

(IV)

$R_6$ is hydrogen, methyl, ethyl or phenyl,
$R_7$ is hydrogen, $-OR_{10}$ or $-N(R_{11})(R_{12})$, and $R_{10}$ is alkyl of 1 to 18 carbon atoms, alkenyl of 3 to 4 carbon atoms, benzyl, 2-cyanoethyl, an aliphatic, cycloaliphatic, aromatic or araliphatic acyl group of up to 18 carbon atoms which can be substituted in the aromatic moiety by halogen, alkoxy of 1 to 4 carbon atoms, alkyl of 1 to 4 carbon atoms and/or hydroxy, $R_{11}$ is alkyl of 1 to 18 carbon atoms, alkenyl of 3 to 4 carbon atoms or phenylalkyl of 7 to 9 carbon atoms, and $R_{12}$ has the same meaning as $R_{11}$ or is an aliphatic, cycloaliphatic, aromatic or araliphatic acyl group of up to 18 carbon atoms which can be substituted in the aromatic moiety by halogen, alkoxy of 1 to 4 carbon atoms, alkyl of 1 to 4 carbon atoms and/or hydroxyl,

or a salt thereof.

2. A malonic acid derivative according to claim 1, wherein

$R_1$ is hydrogen or methyl,
$R_2$ is $C_1-C_8$alkyl, allyl, methallyl, benzyl or phenyl,

$R_3$ is $C_2$—$C_8$alkyl, allyl, methallyl or benzyl,
Z is a group of the formula II or III,
$R_4$ is hydrogen, $O^{\cdot}$, alkyl of 1 to 8 carbon atoms, allyl, propargyl, benzyl, formyl acetyl, acryloyl or crotonyl,
$R_5$ is hydrogen, alkyl of 1 to 12 carbon atoms, cyclohexyl, benzyl or phenyl,
$R_6$ is hydrogen, methyl or ethyl,
$R_7$ is hydrogen or $-OR_{10}$ and $R_{10}$ is hydrogen, alkyl of 1 to 6 carbon atoms, alkanoyl of 2 to 12 carbon atoms, benzoyl or benzyl.

3. A malonic acid derivative according to claim 1, wherein

| | |
|---|---|
| $R_1$ | is hydrogen or methyl, |
| $R_2$ | is $C_1$—$C_8$alkyl, allyl, methallyl, benzyl or phenyl, |
| $R_3$ | is $C_2$—$C_8$alkyl or benzyl, |
| Z | is a group of the formula II or III, |
| $R_4$ | is hydrogen, methyl, benzyl or acetyl, |
| $R_5$ | is hydrogen or $C_1$—$C_4$alkyl, and |
| $R_6$ and $R_7$ | are hydrogen. |

4. A malonic acid derivated according to claim 1, wherein

| | |
|---|---|
| $R_1$ | is hydrogen or methyl, |
| $R_2$ | is $C_1$—$C_6$alkyl, allyl, methallyl or benzyl, |
| $R_3$ | is benzyl, and |
| Z | is a group of the formula II or III, |
| $R_4$ | is hydrogen or methyl, and |
| $R_5$, $R_6$ and $R_7$ | are hydrogen. |

5. A malonic acid derivative according to claim 1, which is bis-[2-(2,2,6,6-tetramethyl-1-piperidinyl)-ethyl]dibenzylmalonate.

6. Stabilised organic material which contains one of the malonic acid derivatives claimed in any one of claims 1 to 5.

7. Stabilised organic material according to claim 6 which is a polyolefin, styrene polymer or polyurethane.

8. A process for stabilising organic material, characterised in that a malonic acid derivative according to any one of claims 1 to 5 is used.

9. A process according to claim 1 for the production of a malonic acid derivative or a salt thereof according to any one of claims 1 to 5, characterised in that $R_2$ and/or $R_3$ is introduced into a malonic acid derivative of the formula I, in which $R_2$ and/or $R_3$ is hydrogen, in a manner which is in itself known, or, in a $R_2$—$R_3$-malonic acid di-lower alkyl ester, lower alkoxy is exchanged for the radical Z in a manner which is in itself known, and, if desired, a substituent $R_4$ is introduced into a malonic acid derivative of the formula I, in which $R_4$ is hydrogen, and/or a free compound is converted into a salt, a salt into a free compound or a salt into another salt.

**Revendications**

1. Dérivés de l'acide malonique répondant à la formule (I) et leurs sels:

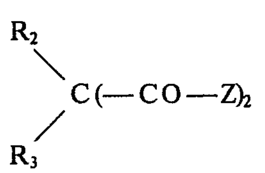

(I)

formule dans laquelle

Z représente un radical répondant à l'une des formules (II) et (III)

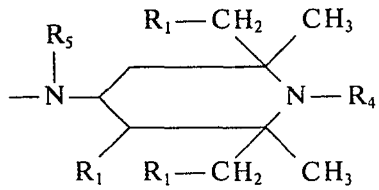

(II)

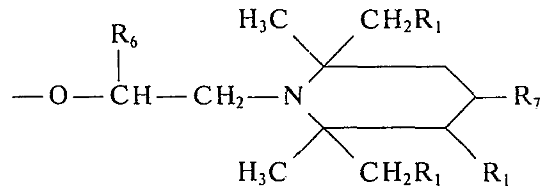

(III)

$R_1$ représente l'hydrogène ou un alkyle en $C_1-C_4$,
$R_2$ représente un alkyle en $C_1-C_{12}$, un alcényle en $C_3$ ou $C_4$, un benzyle, un phényle éventuellement porteur d'un alkyle en $C_1-C_8$ ou d'un alcoxy en $C_1-C_8$, ou un cyano,
$R_3$ représente un alkyle en $C_1-C_{12}$, un alcényle en $C_3$ ou $C_4$ ou un benzyle,
$R_4$ représente l'hydrogène, $O^\cdot$, $-OH$, un alkyle en $C_1-C_{12}$, un alcényle en $C_3$ ou $C_4$, un propargyle, un benzyle ou un radical $-CH_2-CH(OR_9)-R_8$ dans lequel $R_8$ représente l'hydrogène, un méthyle ou un phényle et $R_9$ l'hydrogène ou un radical $A-CO-$; ou $R_4$ représente un radical $A-CO-$ et, dans les deux cas, A représente un alkyle en $C_1-C_{18}$, un alcényle en $C_2$ ou $C_3$, un cyclohexyle, un phényle, un benzyle, un radical phényle, phényl-méthyle ou phényl-éthyle porteur de 2 alkyles contenant chacun de 1 à 4 atomes de carbone et d'un groupe hydroxy, un alkylamino en $C_1-C_{12}$, un dialkylamino en $C_2-C_{16}$, un anilino, un alcoxy en $C_1-C_{12}$, un benzyloxy ou un phénoxy,
$R_5$ représente l'hydrogène, un alkyle en $C_1-C_{18}$, un alcényle en $C_3$ ou $C_4$, un propargyle, un cycloalkyle en $C_5$ à $C_{12}$, un aralkyle en $C_7$ à $C_{14}$ éventuellement porteur d'un OH, ou un radical de formule (IV)

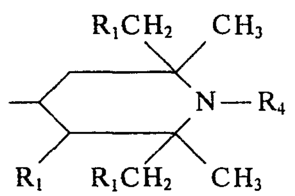

(IV)

$R_6$ représente l'hydrogène, un méthyle, un éthyle ou un phényle,
$R_7$ représente l'hydrogène, un groupe $-OR_{10}$ ou un groupe $-N(R_{11})(R_{12})$ dans lesquels $R_{10}$ représente un alkyle en $C_1-C_{18}$, un alcényle en $C_3$ ou $C_3$, un benzyle, un cyano-2 éthyle, un radical acyle aliphatique, cycloaliphatique, aromatique ou araliphatique contenant au plus 18 atoms de carbone, éventuellement porteur, sur sa partie aromatique, d'un halogène, d'un alcoxy en $C_1-C_4$, d'un alkyle en $C_1-C_4$ et/ou d'un hydroxy, $R_{11}$ représente un alkyle en $C_1-C_{18}$, un alcényle en $C_3$ ou $C_4$ ou un phénylalkyle en $C_7-C_9$, et $R_{12}$ a la même signification que $R_{11}$ ou représente un radical acyle aliphatique, cycloaliphatique, aromatique, ou araliphatique contenant au plus 18 atomes de carbone, éventuellement porteur, sur sa partie aromatique, d'un halogène, d'un alcoxy en $C_1-C_4$, d'un alkyle en $C_1-C_4$ et/ou d'un hydroxy.

2. Dérivés de l'acide malonique selon la revendication 1, dans lesquels:

$R_1$ représente l'hydrogène ou un méthyle,
$R_2$ représente un alkyle en $C_1-C_8$, un allyle, un méthallyle, un benzyle ou un phényle,
$R_3$ représente un alkyle en $C_2-C_8$, un allyle, un méthyllyle ou un benzyle,
Z représente un radical de formule (II) ou (III),
$R_4$ représente l'hydrogène, $O^\cdot$, un alkyle en $C_1-C_8$, un allyle, un propargyle, un benzyle, un formyle, un acétyle, un acryloyle ou un crotonoyle,
$R_5$ représente l'hydrogène, un alkyle en $C_1-C_{12}$, un cyclohexyle, un benzyle ou un phényle,
$R_6$ représente l'hydrogène, un méthyle ou un éthyle, et
$R_7$ représente l'hydrogène ou un groupe $-OR_{10}$ dans lequel $R_{10}$ désigne un alkyle en $C_1-C_6$, un alcanoyle en $C_2-C_{12}$, un benzoyle ou un benzyle.

3. Dérivés de l'acide malonique selon la revendication 1, dans lesquels:

$R_1$ représente l'hydrogène ou un méthyle,
$R_2$ représente un alkyle en $C_1-C_8$, un allyle, un méthallyle, un benzyle ou un phényle,
$R_3$ représente un alkyle en $C_2-C_8$ ou un benzyle,
Z représente un radical de formule (II) ou (III),.
$R_4$ représente l'hydrogène, un méthyle, un benzyle ou un acétyle,

| | |
|---|---|
| $R_5$ | représente l'hydrogène ou un alkyle en $C_1-C_4$, et |
| $R_6$ et $R_7$ | représentent chacun l'hydrogène. |

4. Dérivés de l'acide malonique selon la revendication 1, dans lesquels:

| | |
|---|---|
| $R_1$ | représente l'hydrogène ou un méthyle, |
| $R_2$ | représente un alkyle en $C_1-C_6$, un allyle, un méthallyle ou un benzyle, |
| $R_3$ | représente un benzyle, |
| Z | représente un radical de formule (II) ou (III), |
| $R_4$ | représente l'hydrogène ou un méthyle, et |
| $R_5$, $R_6$ et $R_7$ | représente chacun l'hydrogène. |

5. Dérivé de l'acide malonique selon la revendication 1, en l'espèce le dibenzyl-malonate de bis-[(tétraméthyl-2,2,6,6 pipéridyl-1)-2 éthyle].

6. Matière organique stabilisée qui contient l'un des dérivés de l'acide malonique cités à l'une quelconque des revendications 1 à 5.

7. Matière organique stabilisée selon la revendication 6, matière qui est une polyoléfine, un polymère de styrène ou un polyuréthanne.

8. Procédé pour stabiliser une matière organique, caractérisé en ce qu'on utilise un dérivé de l'acide malonique selon l'une quelconque des revendications 1 à 5.

9. Procédé selon la revendication 1 pour la préparation de dérivés de l'acide malonique ou de sels de ceux-ci selon l'un quelconque des revendications 1 à 5, procédé caractérisé en ce qu'on introduit $R_2$ et/ou $R_3$, de manière connue, dans un dérivé de l'acide malonique de formule (I) dans lequel $R_2$ et/ou $R_3$ représentent l'hydrogène, ou on échange les radicaux alcoxy inférieurs contenus dans un malonate de dialkyle inférieur porteur de $R_2$ et $R_3$, de manière connue, contre des radicaux Z, et, si on le désire, on introduit, dans un dérivé de l'acide malonique de formule (I) dans lequel $R_4$ représente l'hydrogène, de manière connue, un substituant $R_4$, et/ou on transforme un composé libre en un sel, un sel en un composé libre ou un sel en un autre sel.